# EUROPEAN PATENT APPLICATION

(11) **EP 4 686 422 A1**
(43) Date of publication of application: **04.02.2026**
(21) Application number: 25191620.1
(22) Date of filing: 24.07.2025
(51) Int. Cl.: A24F 23/02, A24B 13/00, A61K 9/00, B65D 85/808

(54) **PRODUCT FOR ORAL DELIVERY AND MANUFACTURE THEREOF**

(30) Priority: 01.08.2024 GB 202411360; 09.01.2025 GB 202500252
(71) Applicant: Nonwovenn Ltd, Bridgwater, Somerset TA6 4NZ (GB)
(72) Inventor: CLARKE, Jordan, Somerset, TA6 4NZ (GB); THOMAS-HILLMAN, Leuan, Somerset, TA6 4NZ (GB); HILL, Dave, Somerset, TA6 4NZ (GB); HUTCHINSON, Jeff, Somerset, TA6 4NZ (GB)
(74) Representative: Mewburn Ellis LLP

(57) **Abstract**

A method of manufacturing a product for oral delivery of an active substance is provided. The product comprises a consolidated fabric and the active substance. The method comprises the steps of: providing a fibre web; consolidating the fibre web to provide the consolidated fabric; heating the consolidated fabric; allowing the consolidated fabric to cool; and subsequently, applying a binder containing the active substance to the consolidated fabric. A product for oral delivery of an active substance is also provided. The product comprises a chemically bonded fabric. The chemically bonded fabric comprises: a first binder; and a second binder containing the active substance.

## Description

This application claims priority from GB2411360.7 filed 01/08/2024, the contents and elements of which are herein incorporated by reference for all purposes.

This application claims priority from GB2500252.8 filed 09/01/2025, the contents and elements of which are herein incorporated by reference for all purposes.

### Field of the Invention

The present invention relates to a method of manufacturing a product for oral delivery of an active substance and a product for oral delivery of an active substance.

### Background

It is known to use fabrics (e.g. non-woven fabrics) to manufacture products for oral delivery of a substance comprising an active substance (e.g. flavour compounds). For example, fabrics are used to make pouches for containing an individual portion of a product, such as smokeless tobacco (also known as "snus"), coffee, tea, etc. from which an active substance (e.g. nicotine, caffeine, and/or flavour compounds) is to be extracted. Examples of oral pouched products formed from a non-woven fabric can be found in US 2014/0026912 A1 and US 2012/0103353 A1.

Products comprising structures other than pouches are known, for example EP4117460A1 providing a chewable product for oral delivery of a substance having a non-woven fabric substrate having a matrix of interleaved fibres and a substance held within the matrix.

Release of the active substance to the user of such products is dependent on, amongst other factors, the amount of active substance contained in the product, the ability of fluid to reach the active substance, and the surface area of the active substance in contact with fluid.

Active substances, in particular flavour compounds, are often highly volatile, and various measures have been taken to improve retention of active substances on products for oral delivery.

The present invention has been devised in light of the above considerations.

### Summary of the Invention

In a first aspect there is provided a method of manufacturing a product for oral delivery of an active substance. The product comprises a consolidated fabric and the active substance. The method comprises the steps of: providing a fibre web; consolidating the fibre web to provide the consolidated fabric; heating the consolidated fabric; allowing the consolidated fabric to cool; and subsequently, applying a binder containing the active substance (also referred to as an 'active-substance binder') to the consolidated fabric.

In contrast to conventional pouched products for oral delivery of substances, where the active substance is contained within a pouch composition within a cavity defined by the fabric pouch, the present invention provides the active substance on/in the base fabric (e.g. the fabric used to provide the pouched product) itself, thereby reducing the distance that the active substance has to diffuse through the product and the user's oral cavity, which can provide the user with a more immediate flavour hit.

Due to the volatility of active substances, in particular flavourants, the active substance is preferably provided in/on the base fabric in such a way that the active substance does not readily volatilise, either during manufacture or during subsequent storage of the product. Incorporating the active substance into the binder reduces the rate of volatilisation of the active substance during storage. However, typically during manufacture of a fabric (e.g. a chemically bonded fabric), heating of the fabric is conducted after consolidating the fabric in order to thermally induce a structural change in the fabric, e.g. to dry the fabric (e.g. drive off any solvent used in forming the fibre web, or that is otherwise present in the fabric) and/or to cure and set a binder applied to the fibre web during consolidation.

This heating step can increase volatilisation of any active substance contained in the fabric at that stage, due to the elevated temperatures the fabric is heated to during drying and/or curing. Thus, in the present invention, the consolidated fabric is heated, and then, after heating and allowing the fabric to cool, a binder containing the active substance is applied. By applying a binder after consolidating and heating the fabric to dry and/or cure it, it is possible to incorporate the active substance within a binder without having to subsequently heat the active substance to dry and/or cure the fabric, or with less heating of the active substance to dry and/or cure the fabric than a method where a binder containing the active substance was applied to the consolidated fabric prior to drying and/or curing.

Finally, by allowing the consolidated fabric to cool prior to applying the binder containing the active substance, it is possible to reduce the wastage of the active substance; for a given final amount of the active substance contained in the product, less active substance needs to be applied during manufacture than if no time for cooling was provided, as less active substance will be volatilised off during manufacture.

Consolidating the fibre web may be defined as the process of converting the fibre web into a fabric by securing the fibres together (e.g. through mechanical entangling of fibres, through the thermal bonding of fibres to each other, and/or through the binding of fibres together by a binder), as the skilled person would understand from their common general knowledge.

The step of consolidating the fibre web may comprise applying a first binder to the fibre web; in this way, the consolidated fabric is a chemically bonded fabric. The first binder applied to consolidate the fibre web may be applied as a first binder solution. The first binder solution may comprise a high proportion of solvent (e.g. water), and thus requires extensive heating to dry out the resulting chemically bonded fabric, making the present method particularly suitable. Additionally, the method may comprise heating the fabric in excess of the boiling temperature of the solvent to cure the first binder.

The step of heating the consolidated fabric may be to both dry the consolidated fabric and cure and/or set the first binder (i.e. inducing chemical reactions (e.g. cross linking) within the first binder to toughen, harden, and/or solidify the first binder).

Alternatively, the step of consolidating the fibre web may comprise hydroentangling the fibre web. This may also introduce moisture into the fabric and hence heating may be used to dry the consolidated fabric.

Alternatively, or additionally, to where the step of consolidation introduces moisture into the fabric, the step of providing the fibre web may itself comprise a process resulting in a fibre web that is not dry. By way of example, fibre webs provided by melt blowing or wet laying processes may result in fibre webs having a moisture content greater than zero (e.g. greater than the equilibrium moisture content with the ambient air, typically about 10% by mass). In such a case, it is still advantageous for the method of manufacturing to comprise heating the consolidated fabric (e.g. to dry the consolidated fabric).

By applying the first binder to the fabric and subsequently applying the binder containing the active substance (also referred to as the 'active-substance binder' or 'second binder') to the chemically bonded fabric, the first binder and second binder may provide a core and shell structure on the fibres and/or in spaces between fibres (e.g. to create a core and shell structure in a webbed space between adjacent fibres). In particular, the second binder may be provided on an outer surface of the fabric, where the active substance it contains can be more readily reached by, and dissolved into, the user's saliva, whilst the first binder, having been at least partially coated/covered by the second binder, is in reduced contact with the user's saliva. For a given amount of active substance, this can provide faster transfer of active substance to the user in comparison to if the active substance was contained solely in the first binder or was contained in both the first binder and the second binder (e.g. if one had a core-shell structure in which the first binder and second binder contained active substance).

The first binder may be applied to the fibre web within a first binder solution. The first binder solution may comprise a first binder (i.e. a fibre-binding adhesive) and a first solvent. The second binder (i.e. a fibre-binding adhesive) may be applied within a second binder solution. The second binder solution may comprise the second binder and a second solvent. The first binder may be different to the second binder. The first solvent may be different to, or the same as, the second solvent. The first solvent and/or the second solvent may be water. The first binder and/or first binder solution may not comprise an active substance. The first binder may be flavourless (e.g. the first binder may not comprise a flavour compound). The second binder solution may have a lower solvent content (e.g. by mass) than the first binder solution. In this way, any heating conducted after applying the second binder solution (e.g. to dry the fabric) may comprise applying less heat energy to the fabric than step of heating the consolidated fabric prior to applying the second binder. Alternatively, the second solvent may evaporate naturally (i.e. without a further heating step).

The binder containing the active substance may be a hydrophilic binder, e.g. a hydrophilic acrylic binder, a hydrophilic biodegradable binder compound such as polybutylene succinate (PBS); or a hydrophilic polyurethane. This provides a convenient transfer mechanism through which to transport the active substance from the binder to the user by wetting the binder and dissolving the active substance contained in the binder. The first binder may be a hydrophilic binder or a hydrophobic binder, for example, a (hydrophilic) acrylic binder. The hydrophilic/hydrophobic nature of the first binder may not be important to active substance delivery where the first binder does not contain an active substance.

The principal role of the binder containing the active substance is as a carrier for the active substance. Accordingly, the binder containing the active substance may only be applied to a proportion of the outer surface of the consolidated fabric, for example, said binder may be applied to 40% or less of the area of the outer surface of the consolidated fabric (e.g. about 30% of the area of the outer surface of the consolidated fabric). This can provide for dosage control of the active substance and also may mean that portions of the outer surface of the consolidated fabric can still be heat sealed (e.g. if the binder containing the active substance is not heat-sealable). Herein, "chemically bonded" may mean a web of staple fibres that are secured together by adhesive bonding of fibres, i.e. by a binder that acts to physically interconnect the staple fibres and provide the fabric with increased strength and durability through a combination of mechanical interlinking, cohesiveness to itself and intermolecular forces of attraction.

The active-substance binder may have a contact angle less than or equal to 90° and greater than or equal to 0°. Having such a contact angle and/or water permeability provides a binder that is easily wettable by a user's saliva. The contact angle may be measured according to ASTM D5946.

The first binder and/or the active-substance binder may be a non-fibrous binder. As the product is intended for oral use, the first binder and/or active-substance binder is preferably a food-contact-grade substance, and more preferably a food-grade substance.

The fibre web may comprise one or more types of fibres (e.g. staple fibres). The types of fibres may be selected from the group consisting of natural fibres, such as cotton and/or bamboo; regenerated cellulosic fibres, such as viscose, Modal and/or lyocell; and thermoplastic fibres, such as polypropylene, polyethylene, polyester, ethylene vinyl acetate, polyurethane, polylactic acid and polyamides. As the product is intended for oral use, the fibres are preferably food-contact-grade, and more preferably food-grade.

The active substance may be a pharmaceutical material, smokeless tobacco, nicotine salts, free nicotine base, cannabidiol, coffee, tea, flavouring or the like. For example, the active substance may consist or comprise any one or more of: amino acids, in particular taurine, lysine, tryptophane, theanine, tyrosine, or 5-HTP; alkaloids, in particular nicotine, anatabine, caffeine, theobromine or theophylline; natural or synthetic cannabinoids, in particular cannabinol, cannabidivarin, cannabichromen, cannabidiol, cannabigerol, tetrahydrocannabivarin, Delta-8-tetrahydrocannabinol, Delta-9-tetrahydrocannabinol or Delta-x-tetrahydro cannabidiol; hormones, in particular melatonin; vitamins or minerals. The active substance may comprise one or more compounds selected from the group consisting of: Nicotine; Caffeine; Cannabidiol; Ethyl acetate; Benzyl acetate; Ethyl butyrate; Isoamyl acetate; Citral; Benzaldehyde; Cinnamaldehyde; Alpha-GPC; Salidroside; Uridine Monophosphate; L-theanine; Methyl salicylate; Menthol; Menthone; Carvone; N,2,3-trimethyl-2-propan-2-yl butanamide; Neryl Acetate; Octyl acetate; and Limonene.

The first binder may be applied to the fibre web via a variety of methods, for example, printing the first binder onto the fibre web; spraying the first binder solution onto the fibre web; foam bonding the fibre web with the first binder solution; knife-over-roll coating the first binder onto the fibre web; depositing (e.g. layering, scattering, or spraying) solid first binder onto the fibre web and melting the solid first binder; or immersing the fibre web in the first binder solution.

The step of heating the consolidated fabric may comprise heating the consolidated fabric to a temperature greater than or equal to the boiling point or the flash point of the active substance. Because, in the present invention, the active substance is only applied to the consolidated fabric subsequent to heating the consolidated fabric to dry and/or cure the consolidated fabric and allowing it to cool, the consolidated fabric can be heated to in excess of the boiling point or flash point of the active substance to rapidly dry and/or cure the consolidated fabric without impacting the loading of active substance onto the consolidated fabric. The step of heating the consolidated fabric may comprise heating the consolidated fabric to a temperature greater than or equal to 100°C, greater than or equal to 120°C, or greater than or equal to 140°C. The step of heating the consolidated fabric may comprise heating the consolidated fabric to a temperature less than or equal to 180°C, or less than or equal to 160°C. The boiling point of the active substance may be measured according to ISO 4626:2023 (e.g. as the initial boiling point or dry point according to ISO 4626:2023). The flash point of the active substance may be measured according to Procedure A or B in ISO 3679:2022.

The step of heating the consolidated fabric may be conducted such that the first solvent content / moisture content in the consolidated fabric is less than or equal to 0.1 g solvent (e.g. first solvent) / g dry mass, e.g. prior to applying the binder containing the active substance. The step of heating the consolidated fabric may be conducted such that the first solvent /moisture content in the consolidated fabric is substantially zero prior to applying the active-substance binder (e.g. immediately after finishing heating the consolidated fabric). As ambient air contains water vapour, the consolidated fabric may then take up water after heating as it equilibrates with the ambient air, typically this amounts to about 10% by mass. The step of heating the consolidated fabric may be conducted such that the first solvent / moisture content in the consolidated fabric after heating is less than or equal to the equilibrium content of water in the fabric as the fabric equilibrates with the water content in the ambient air.

Properly drying the consolidated fabric avoids degradation of the fabric during storage and subsequent conversion into a product for oral delivery (e.g. to avoid bacterial growth, to avoid the fabric having an odour, to avoid the discolouration of the fabric, to avoid the fabric developing an unpleasant taste, and/or to improve the handling and processability of the fabric in subsequent steps in the manufacture of a product for oral delivery). Moreover, where the consolidated fabric is a chemically bonded fabric and the first binder is cured and/or set by heating the chemically bonded fabric, this typically involves heating the chemically bonded fabric to drive off all of the first solvent and then further heating the fabric to induce chemical reactions within the first binder, and thereby toughen, harden, and/or solidify the first binder.

The step of allowing the consolidated fabric to cool may be conducted such that the temperature of the consolidated fabric is less than or equal to 70°C, less than or equal to 60°C, less than or equal to 45°C, or preferably less than or equal to 40°C. The step of allowing the consolidated fabric to cool may be conducted such that the temperature of the consolidated fabric is greater than or equal to 20°C (e.g. room temperature). In this way, the binder containing the active substance solution is only applied to the consolidated fabric once any residual heat from drying and/or curing the consolidated fabric has dissipated. In particular, the step of allowing the consolidated fabric to cool may be conducted such that the temperature of the consolidated fabric is less than the boiling point of the active substance contained in the active-substance binder, or more preferably such that the temperature of the consolidated fabric is less than the flash point of the active substance contained in the active-substance binder. In this way, the amount of active substance volatilised off when applying the active-substance binder to the consolidated fabric can be reduced.

In some examples, the step of allowing the consolidated fabric to cool may be conducted by passive cooling (e.g. radiative and natural convective cooling). However, the step of allowing the consolidated fabric to cool may alternatively be conducted by active cooling (e.g. by forced convection or conduction). Active cooling may provide increased cooling rates compared to passive cooling, providing a greater temperature reduction of the consolidated fabric and/or a allowing a greater throughput of consolidated fabric through the method in comparison to passive cooling. Active cooling may be provided, for example, by impinging an airflow onto the consolidated fabric, or by passing the fabric over one or more cooled rollers / drums.

The step of applying the active-substance binder may comprise applying said binder to the consolidated fabric when the temperature of the consolidated fabric is less than or equal to 75°C. That is, the 'application temperature' for the active-substance binder to the consolidated fabric may be less than or equal to 75°C. The application temperature for the active-substance binder may be less than or equal to 60°C, less than or equal to 45°C, or preferably less than or equal to 40°C. The application temperature for the active-substance binder may be greater than or equal to 20°C (e.g. room temperature), e.g. greater than or equal to 20°C and less than or equal to 75°C. In some examples, the application temperature for the active-substance binder may be less than or equal to 40°C and greater than or equal to 30°C. In this way, the amount of active substance volatilised off when applying the active-substance binder to the consolidated fabric can be reduced. It may be advantageous for the application temperature of the active-substance binder to be an elevated temperature (e.g. above room temperature) to assist in spreading of the active-substance binder on the consolidated fabric and evaporation of any second solvent in a binder solution that the active-substance binder is applied with. Where the step of allowing the consolidated fabric to cool is conducted such that the temperature of the consolidated fabric is below the application temperature for the active-substance binder, the method may further comprise re-heating the consolidated fabric (e.g. to the application temperature for the binder).

The product may be a pouched oral product, the consolidated fabric providing a pouch defining a cavity. The method may further comprise: forming the pouch from the consolidated fabric; and filling the cavity with a pouch filling. The pouch filling may comprise a further active substance. The active substance and the further active substance may be different active substances (and, optionally, different types of active substance (e.g. a flavour compound and a pharmaceutical compound), for example, the active substance provided in the binder may be incompatible with the further active substance included in the pouch filling. Providing a further active substance in the pouch filling in addition to that provided in the binder can allow the release characteristics of the different active substances to be controlled, e.g. the further active substance contained in the pouch filling would be expected to take longer to reach the user than the active substance contained in the binder on the fabric. This is because the active substance contained in the binder will enter solution in the user's saliva earlier, and has a shorter distance to diffuse within the user's oral cavity, than the further active substance contained in the pouch filling. By way of further example, the active substance may comprise a first flavour compound and the further active substance may comprise a second, different flavour compound. Providing the first flavour compound and second flavour compound in the pouched oral product in this manner can provide a more complex flavour profile to the user. Alternatively, the product may be a non-pouched oral product.

The further active substance may be a pharmaceutical material, smokeless tobacco, nicotine salts, free nicotine base, cannabidiol, coffee, tea, flavouring or the like. For example, the further active substance may consist or comprise any one or more of: amino acids, in particular taurine, lysine, tryptophane, theanine, tyrosine, or 5-HTP; alkaloids, in particular nicotine, anatabine, caffeine, theobromine or theophylline; natural or synthetic cannabinoids, in particular cannabinol, cannabidivarin, cannabichromen, cannabidiol, cannabigerol, tetrahydrocannabivarin, Delta-8-tetrahydrocannabinol, Delta-9-tetrahydrocannabinol or Delta-x-tetrahydro cannabidiol; hormones, in particular melatonin; vitamins or minerals. The further active substance may comprise one or more compounds selected from the group consisting of: Nicotine; Caffeine; Cannabidiol; Ethyl acetate; Benzyl acetate; Ethyl butyrate; Isoamyl acetate; Citral; Benzaldehyde; Cinnamaldehyde; Alpha-GPC; Salidroside; Uridine Monophosphate; L-theanine; Methyl salicylate; Menthol; Menthone; Carvone; N,2,3-trimethyl-2-propan-2-yl butanamide; Neryl Acetate; Octyl acetate; and Limonene.

The active-substance binder may be applied to the consolidated fabric via a variety of methods, for example, printing the active-substance binder onto the consolidated fabric; spraying the active-substance binder onto the consolidated fabric; foam bonding the consolidated fabric with the active-substance binder; immersing the consolidated fabric in the active-substance binder); knife-over-roll coating the active-substance binder onto the consolidated fabric; or depositing the active-substance binder onto the consolidated fabric as a solid and melting said binder. Foam bonding the consolidated fabric may be preferred compared to spraying the active-substance binder onto the consolidated fabric or immersing the consolidated fabric in the active-substance binder, as foam bonding results in a reduced second solvent content in the consolidated fabric compared to the other processes of applying the active-substance binder. Foam bonding may be defined as a method for applying a binder to the fibre web to bind the fibres, in which the binder solution is foamed (i.e. comprises a gas phase dispersed in the liquid binder solution phase) when it is contacted with the fibre web to coat the fibres (as defined by the Association of the Nonwoven Fabrics Industry (INDA) - www.inda.org/about-nonwovens/nonwovens-glossary-of-terms/). Printing the active-substance binder onto the consolidated fabric may comprise adhering the active-substance binder to the consolidated fabric in the form of discrete dots (e.g. by screen printing the binder onto the consolidated fabric).

Where the active-substance binder is printed onto the consolidated fabric, the viscosity of the active-substance binder may be greater than or equal to 19 cPas and less than or equal to 30 cPas. Preferably, the viscosity may be around 25 cPas, e.g. between 24 cPas and 26 cPas. Such viscosities are suitable for (screen) printing of said substance or first adhesive, particularly in the form of discrete dots such that the dots do not flow and amass after printing. Depending on the composition of the compounds in said first adhesive or substance (e.g. a binder compound, such as PBS), said first adhesive or substance may further comprise a thickening agent in order to provide the first adhesive or substance with such a viscosity. The thickening agent may be selected from the group consisting of: kappa-carrageenan (Kappa-C), sulphated polysaccharides (SP), Hydroxypropyl Methylcellulose (HPMC)). Such thickening agents are food-contact safe and thus suitable for inclusion in products for oral delivery of a substance.

Where the product is a pouched oral product, the step of applying the active-substance binder to the consolidated fabric may be conducted before the step of forming the pouch. This facilitates applying the active-substance binder to the consolidated fabric via a variety of methods (e.g. printing, spraying, foam bonding, knife-over-roll coating, or immersing). Alternatively, the step of applying the active-substance binder to the consolidated fabric may be conducted after the step of forming the pouch. In such a case, the active-substance binder may be applied to the consolidated fabric by printing the active-substance binder onto the pouch; or spraying the active-substance binder onto the pouch.

The consolidated fabric may be a substantially planar structure, having a first side opposite a second side. The active-substance binder may be applied to only one of said first and second sides e.g. sprayed onto a single side of the consolidated fabric or printed onto a single side of the consolidated fabric. This is particularly relevant where the consolidated fabric is to be applied within a pouched oral product, or other cases where the product is configured such that a single side of the consolidated fabric is exposed on an exterior of the product, since then the active-substance binder may be applied to only the surface that will form an exterior of the product. In this way, the active substance may be provided only on the side of the consolidated fabric that comes into contact with a user's saliva first. This can increase the speed of transfer of the active substance to the user relative to the amount of active-substance binder applied to the consolidated fabric.

The method may further comprise drying (e.g. air drying) the consolidated fabric after applying the active-substance binder. In some embodiments, the method may further comprise heating the consolidated fabric after applying the active-substance binder. This heating step can remove any moisture / solvent added to the consolidated fabric by the application of active-substance binder. In heating the consolidated fabric after applying the active-substance binder, the consolidated fabric may be heated to a temperature less than or equal to 70° C, less than or equal to 60°C, less than or equal to 45°C, or preferably less than or equal to 40°C. Preferably, the consolidated fabric is heated to a temperature that is less than the flash point of the active substance. This can provide a balance between drying the consolidated fabric after applying the active-substance binder, and reducing the amount of active substance volatilised off by the heating step.

The moisture content of the consolidated fabric after applying the active-substance binder (e.g. after drying the consolidated fabric after applying said binder) may be less than or equal to 0.1 g solvent (e.g. first solvent and/or second solvent) / g dry mass. The solvent content in the consolidated fabric after applying the active-substance binder may be substantially zero. As ambient air contains water vapour, the consolidated fabric may take up water after applying the active-substance binder as it equilibrates with the ambient air, typically this amounts to about 10% by mass. Properly drying the consolidated fabric after applying the active-substance binder avoids degradation of the fabric during storage and subsequent conversion into a product for oral delivery (e.g. to avoid bacterial growth, to avoid the fabric having an odour, to avoid the discolouration of the fabric, to avoid the fabric developing an unpleasant taste, and/or to improve the handling and processability of the fabric in subsequent steps in the manufacture of a product for oral delivery). However, it can be understood that in some cases (e.g. when forming pouched oral products), manufacturing the finished the oral product may involve re-wetting the consolidated fabric (e.g. when adding a pouch filling), but this may be done once the fabric has been processed into the final product form (e.g. after processing a roll of chemically fabric into individual cuts of fabric to form the product).

The consolidated fabric may be a consolidated nonwoven fabric, e.g. a carded nonwoven fabric.

In some embodiments, the active-substance binder may be applied to the consolidated fabric as part of a continuous process, such that the period of time between heating the consolidated fabric and applying the active-substance binder is less than or equal to 5 minutes, preferably less than or equal to 1 minute, more preferably less than or equal to 5 seconds. However, in other embodiments, there may be a significant period of time between heating the consolidated fabric and applying the active-substance binder (e.g. the period of time between heating the consolidated fabric and applying the active-substance binder may be greater than or equal to 30 minutes, or greater than or equal to 1 day). Such a period of time may facilitate passive cooling of the consolidated fabric.

In a second aspect there is provided a product for oral delivery of an active substance, wherein: the product comprises a consolidated fabric that is a chemically bonded fabric; the chemically bonded fabric comprises: a first binder; and a second binder containing the active substance.

At least a portion of the second binder may be disposed on (e.g. overlie) at least a portion of the first binder. In this way at least a portion of the second binder may be disposed on at least a portion of an outer surface of the first binder. By way of example, the first binder and second binder may provide a core and shell structure (e.g. the first binder providing the core, the second binder providing the shell). In this manner, the second binder that comprises the active substance is provided in such a way that the active substance can enter solution in the user's saliva faster than if the active substance was provided in the first binder that is covered by the second binder, because to reach the first binder that the second binder is disposed on, saliva would have to permeate through the second binder. This can then provide faster transfer of the active substance to the user.

The mass of solvent (e.g. first solvent and/or second solvent) in the chemically bonded fabric may be less than or equal to 0.1 g solvent / g dry mass, preferably about 0 g solvent / g dry mass. The mass of binder (e.g. first binder and/or second binder) in the chemically bonded fabric may be greater than or equal to 20 g / g dry mass and/or less than or equal to 60 g / g dry mass, e.g. about 40 g / g dry mass. The mass of the active substance (e.g. nicotine) in the chemically bonded fabric may be greater than or equal to 2 gsm and less than or equal to 12 gsm.

The product may be a pouched oral product, the chemically bonded fabric providing a pouch defining a cavity. The product may comprise a pouch filling situated within the cavity. The pouch filling may comprise a further active substance. In some examples, the active substance and the further active substance may be different active substances (and, optionally, different types of active substance (e.g. a flavour compound and a pharmaceutical compound), for example, the active substance provided in the second binder may be incompatible with the further active substance included in the pouch filling. Providing a further active substance in the pouch filling in addition to that provided in the second binder can allow the release characteristics of the different active substances to be controlled, e.g. the further active substance contained in the pouch filling would be expected to take longer to reach the user than the active substance contained in the second binder on the fabric, since the active substance contained in the second binder will enter solution in the user's saliva earlier, and has a shorter distance to diffuse within the user's oral cavity.

Any one or more of the optional features set out with respect to the first aspect may be applied to the second aspect, except where clearly impermissible or expressly avoided, and are hereby restated in respect of the second aspect.

### Summary of the Figures

Embodiments illustrating the principles of the invention will now be discussed with reference to the accompanying figures in which:
Fig. 1 provides a flowchart for a method of manufacturing a product for oral delivery of an active substance; and
Fig. 2 is a schematic of a manufacturing line for manufacturing a nonwoven product.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

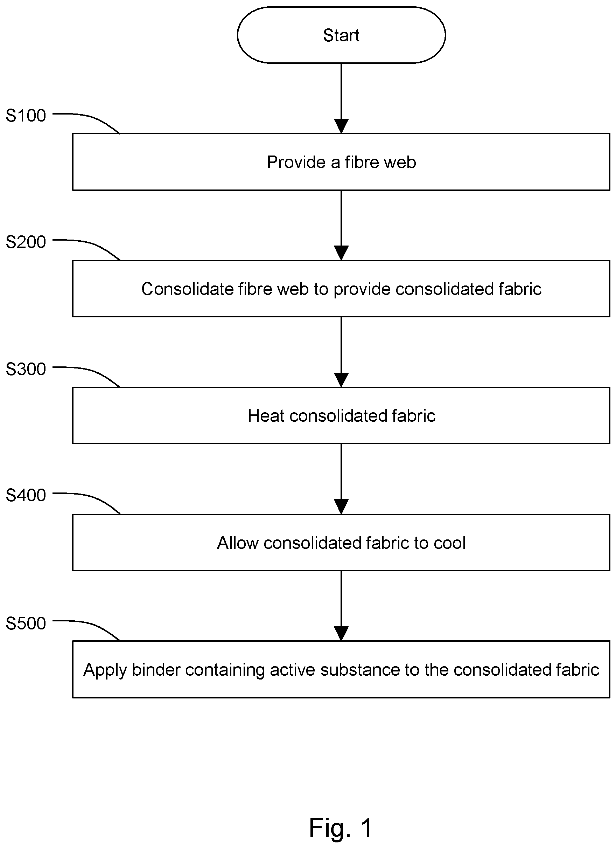

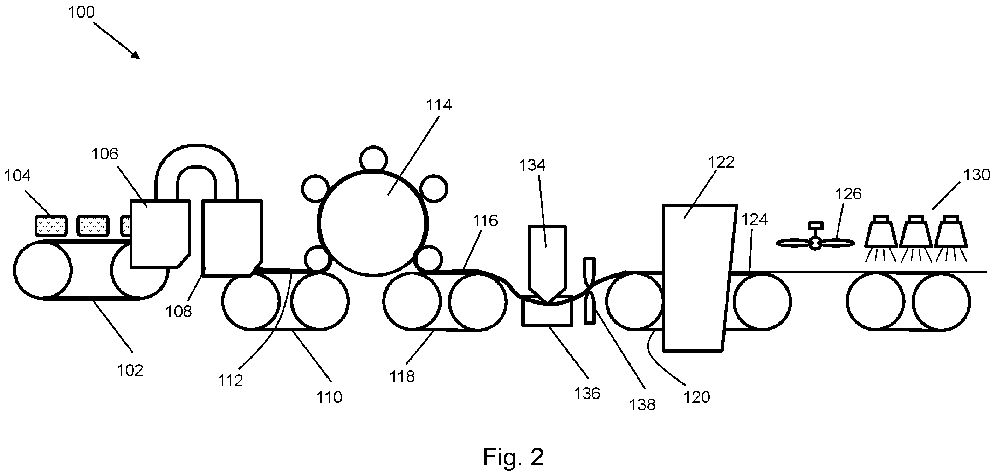

Fig. 1 provides a flowchart for a method of manufacturing a product for oral delivery of an active substance. The flowchart comprises steps S100 to S500. The method in Fig. 1 is discussed below with reference to the schematic of the manufacturing line in Fig. 2.

At step S100 a fibre web is provided. The fibre web comprises a plurality of fibres (e.g. staple fibres) that may be entangled but are not chemically or thermally bonded together.

Step S100 may be conducted in several different ways. In Fig. 2, a first conveyor 102 transports fibre bales 104 to a bale opener 106, which separates and blends the fibres from each bale. The fibre bales 104 comprise staple fibres. In some examples the fibre bales 104 may contain multiple types of staple fibre. The staple fibres in the fibre bales 104 may have any suitable cross-section.

The bale opener 106 is connected to a feed hopper 108 that discharges the blended fibres as a loose fibre web 112 on a second conveyor 110. The loose fibre web 112 is conveyed to a carding machine 114 that combs the web to apply a desired orientation or plurality of orientations to the fibres in the web. The carding machine 114 thus outputs a web 116 on to a third conveyor 118.

In some examples the web 116 may comprise a plurality of carded layers. Each carded layer may be output from a respective carding machine 114 before being combined with the other carded layers into a single web. The plurality of carded layers may be obtained by divided the loose fibre web 112 between the respective carding machines. Providing a plurality of carded layers can improve the uniformity of the web.

The carding process is optional. For example, the fibre web 116 may be formed directly by air laying suitable fibres. In this example, the fibres may be crimped during manufacture to facilitate web formation in an air stream. Alternatively, the fibre web 116 may be formed directly by a wet laying process.

Step S100 terminates following the provision of a fibre web 116.

Subsequently, at step S200, the fibre web is consolidated, in this case by applying a first binder to the fibre web, thereby bonding the plurality of fibres in the fibre web together and providing a consolidated fabric that is a chemically bonded fabric.

Fig. 2 illustrates how step S200 may be conducted by conveying the fibre web 116 using deflector 134 into a pan 136 filled with a first binder solution, so that the first binder solution impregnates or saturates the fibre web 116. The first binder solution comprises the first binder and a first solvent (e.g. water), herein together referred to as the first binder solution. In other examples, the first binder solution may be applied by coating or spraying. The consolidated fibre web 116 is then transported to a fourth conveyor 120 via nip rollers 138, which operate to remove or squeeze excess liquid from the consolidated fibre web 116.

As the fibre web 116 is chemically bonded by the first binder applied using the first binder solution, the chemically bonded fabric provided from step S200 (i.e. reaching the fourth conveyor 120) contains a large quantity of first solvent (e.g. water), even after passing through the nip rollers 138. It is desirable both to dry the fabric and fully cure / stabilise the first binder.

Thus, step S300 in Fig. 1 comprises heating the chemically bonded fabric to dry the chemically bonded fabric and cure and set the first binder. This is illustrated in Fig. 2 by the web 116 being carried through a dryer 122. Typically, the dryer 122 heats the chemically bonded fabric to a temperature between 120°C and 160°C, thereby reducing the first solvent content (e.g. water content) of the chemically bonded fabric to substantially zero and curing and setting the first binder.

Having heated the chemically bonded fabric to dry it (i.e. evaporate the first solvent from the first binder solution) and cure and set the first binder, the method comprises step S400 of allowing the consolidated (chemically bonded) fabric to cool, prior to any subsequent processing taking place. Step S400 may be conducted passively or conducted with active cooling (e.g. forced convection), as is the case in Fig. 2 where the chemically bonded fabric is cooled by a fan 126.

The cooling step S400 is conducted prior to step S500, where a binder containing the active substance (the 'second binder') to be delivered to the user is applied to the chemically bonded fabric The cooling step S400 is conducted so that the amount of the active substance that is volatilised off the fabric after being applied with the second binder at step S500 is reduced. Due to the volatility of active substances, in particular flavourants, applying the active substance to the chemically bonded fabric without the cooling step S400 can result in a substantial amount of the active substance volatilising off the fabric due to the residual heat in the chemically bonded fabric from Step S300. It can be appreciated that the lower the temperature to which the chemically bonded fabric is cooled at step S400, the less active substance will be lost from the chemically bonded fabric during manufacture. Typically, the temperature of the chemically bonded fabric after step S400 is less than or equal to 75°C, and preferably is between 40°C and room temperature (e.g. about 20°C). More generally, step S400 can be conducted such that the temperature of the chemically bonded fabric after step S400 and at the point of applying the second binder in step S500 is less than the boiling point and the flash point of the active substance.

Table 1 provides a list of examples of active substances that may be contained in the second binder and their flash points and boiling points, where applicable.

**Table 1**

| **Active Substance** | **Flash point / °C** | **Boiling point / °C** |
|---|---|---|
| Nicotine | 95 | 247 |
| Caffeine | N/A | 178 |
| Cannabidiol | N/A | 160 |
| Ethyl acetate | -4 | 77.1 |
| Benzyl acetate | 102 | 212 |
| Ethyl butyrate | 26 | 120 |
| Isoamyl acetate | 25 | 142 |
| Citral | 9 | 229 |
| Benzaldehyde | 64 | 178.1 |
| Cinnamaldehyde | 71 | 248 |
| Alpha-GPC | N/A | N/A |
| Salidroside | N/A | N/A |
| Uridine Monophosphate | N/A | N/A |
| L-theanine | N/A | N/A |
| Methyl salicylate | 96 | 222 |
| Menthol | 93 | 215 |
| Menthone | 69 | 207 |
| Carvone | 89 | 231 |
| N,2,3-trimethyl-2-propan-2-yl butanamide | 132 | 233 |
| Neryl Acetate | 99 | 240 |
| Octyl acetate | 83 | 203 |
| Limonene | 50 | 176 |

Step S500 of applying the second binder to the chemically bonded fabric can be conducted via a variety of different methods. Fig. 2 illustrates the second binder being sprayed onto the chemically bonded fabric by a spraying unit 130. This method of applying the second binder can result in the second binder only being applied to one side of the fabric (i.e. the side of the fabric facing the spraying unit 130 in Fig. 2), which can be advantageous in products where this side of the fabric forms an exterior surface of the product, since faster delivery of the active substance may be provided. Alternative methods of applying the second binder include printing (e.g. dot printing) the second binder onto the chemically bonded fabric; foam bonding the chemically bonded fabric with the second binder (e.g. applying a foam of a second binder solution to the chemically bonded fabric); or immersing the chemically bonded fabric in the second binder (e.g. in a second binder solution).

Although Fig. 2 illustrates step S500 being performed immediately following steps S300 and S400, in other embodiments there may be a significant period of time between heating the chemically bonded fabric and applying the second binder. In particular, such a period of time may facilitate passive cooling of the chemically bonded fabric at step S400.

Subsequent to step S500, further steps may be conducted to provide the oral product. For example, in the case of a pouched product, the method may further comprise forming a pouch from the chemically bonded fabric (after the first binder and second binder have been applied), the pouch defining a cavity, and filling the cavity with a pouch filling.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A method of manufacturing a product for oral delivery of an active substance, wherein the product comprises:
a consolidated fabric; and
the active substance; and
wherein the method comprises the steps of:
providing a fibre web;
consolidating the fibre web to provide the consolidated fabric;
heating the consolidated fabric;
allowing the consolidated fabric to cool; and
subsequently, applying a binder containing the active substance to the consolidated fabric.

2. The method according to claim 1, wherein:
consolidating the fibre web comprises applying a first binder to the fibre web;
the consolidated fabric is a chemically bonded fabric; and
optionally, the first binder does not comprise an active substance.

3. The method according to any preceding claim, wherein the step of heating the consolidated fabric comprises heating the consolidated fabric to a temperature greater than or equal to the boiling point of the active substance.

4. The method according to any preceding claim, wherein the step of heating the consolidated fabric is conducted such that the moisture content of the product is less than or equal to 0.1 g solvent / g dry mass.

5. The method according to any preceding claim, wherein:
the product is a pouched oral product, the consolidated fabric providing a pouch defining a cavity;
the method further comprises:
forming the pouch from the consolidated fabric; and
filling the cavity with a pouch filling; and
optionally the step of applying the binder containing the active substance to the consolidated fabric is conducted before the step of forming the pouch.

6. The method according to claim 5, wherein:
the pouch filling comprises a further active substance; and
optionally, the active substance and the further active substance are flavour compounds.

7. The method according to any preceding claim, wherein the step of applying the binder containing the active substance is conducted by:
spraying said binder onto the consolidated fabric;
immersing the consolidated fabric in said binder;
foam bonding the consolidated fabric with said binder;
knife-over-roll coating said binder onto the consolidated fabric;
depositing said binder onto the consolidated fabric as a solid and melting said binder; or
printing said binder onto the consolidated fabric.

8. The method according to any preceding claim, wherein:
the binder containing the active substance is a hydrophilic binder; and/or
the consolidated fabric is a nonwoven fabric.

9. The method according to any preceding claim, wherein:
the consolidated fabric is a substantially planar structure, having a first side opposite a second side; and
the binder containing the active substance is applied to only one of said first and second sides.

10. A product for oral delivery of an active substance, wherein:
the product comprises a consolidated fabric that is a chemically bonded fabric; and
the chemically bonded fabric comprises:
a first binder; and
a second binder containing the active substance.

11. The product according to claim 10, wherein at least a portion of the second binder is disposed on at least a portion of the first binder.

12. The product according to claim 10 or 11, wherein:
the product is a pouched oral product, the chemically bonded fabric providing a pouch defining a cavity;
the product comprises a pouch filling situated within the cavity, the pouch filling comprising a further active substance; and
optionally, the active substance and the further active substance are different.

13. The product according to any one of claims 10 to 12, wherein:
the chemically bonded fabric is a chemically bonded nonwoven fabric and/or
the second binder is a hydrophilic binder.

14. The product according to any one of claims 10 to 13, wherein the first binder does not comprise an active substance.

15. The product according to any one of claims 10 to 14, wherein:
the chemically bonded fabric is a substantially planar structure, having a first side opposite a second side; and
the second binder is applied to only one of said first and second sides.
